(19) Europäisches Patentamt
European Patent Office
Office européen des brevets

(11) EP 1 945 223 B1

(12) **EUROPEAN PATENT SPECIFICATION**

(45) Date of publication and mention of the grant of the patent:
**07.07.2010 Bulletin 2010/27**

(21) Application number: **06851366.2**

(22) Date of filing: **27.10.2006**

(51) Int Cl.:
***A61K 9/08*** *(2006.01)* ***A61K 31/545*** *(2006.01)*

(86) International application number:
**PCT/US2006/041952**

(87) International publication number:
**WO 2007/145656 (21.12.2007 Gazette 2007/51)**

(54) **CEFQUINOME COMPOSITIONS AND METHODS OF THEIR USE**

CEFQUINOMZUSAMMENSETZUNGEN UND VERFAHREN ZU IHRER VERWENDUNG

COMPOSITIONS DE CEFQUINOME ET PROCÉDÉS D'UTILISATION DE CELLES-CI

(84) Designated Contracting States:
**AT BE BG CH CY CZ DE DK EE ES FI FR GB GR HU IE IS IT LI LT LU LV MC NL PL PT RO SE SI SK TR**

(30) Priority: **29.10.2005 US 731463 P**
**13.03.2006 EP 06111029**

(43) Date of publication of application:
**23.07.2008 Bulletin 2008/30**

(73) Proprietor: **Intervet International BV**
**5831 AN Boxmeer (NL)**

(72) Inventors:
- **CADY, Susan M.**
**Yardley, PA 19067 (US)**
- **BARBOT, Carole**
**76520 Boos (FR)**

(74) Representative: **Stumm, Karin et al**
**Intervet International BV**
**Patent Department**
**Wim de Körverstraat 35**
**P.O. Box 31**
**5830 AA Boxmeer (NL)**

(56) References cited:
**EP-A- 0 388 510**
**EP-A1- 0 711 774**
**WO-A-2004/054538**
**GB-A- 1 380 741**
**US-A- 5 747 484**
**US-A1- 2003 186 957**
**US-B2- 6 911 411**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 945 223 B1

## Description

CROSS-REFERENCE TO RELATED PATENT APPLICATIONS

This patent claims priority to U.S. Provisional Patent Application No. 60/731,463 (filed October 29, 2005) and European Patent Application No. 06111029.2 (filed March 13, 2006).

FIELD OF THE INVENTION

This invention is directed generally to cefquinome compositions (including compositions comprising cefquinome salts), processes for making such compositions, uses of such compositions to make medicaments, kits for making such compositions, and methods for using such compositions and kits to treat infections.

BACKGROUND OF THE INVENTION

Cefquinome (CAS no. 84957-30-2) is a β-lactam antibiotic of the cephalosporin class. It has a broad spectrum of activity against both gram-positive and gram-negative bacteria, including *Actinobacillus spp., Actinobacillus pleuropneumoniae, Haemophilus spp., Clostridium spp.*, *Corynebacterium*, *Erysipelothrix rhusiopathiae, Streptococcus spp.,* and *Pasteurella spp.* Cefquinome may, for example, be used to treat meningitis caused by *Streptococcus suis,* epidermatitis caused by *Staphylococcus spp.,* and mastitis-metritis-agalactia syndrome ("MMA") caused by *E. coli* and *Staphylococcus spp.* The shape of the cefquinome molecule tends to facilitate distribution in treated animals and passage through bacterial cell walls, resulting in rapid bactericidal effect following injection. It also tends to be resistant against inactivation by bacteria that produce β-lactamase.

Commercially available formulations of cefquinome have included, for example, COBACTAN 2.5%™, sold by Intervet International B.V., Boxmeer, The Netherlands. COBACTAN 2.5%™, which also has been sold under the name CEPHAGUARD 2.5%™, contains 25 mg/ml of cefquinome sulfate (CAS no. 123766-80-3):

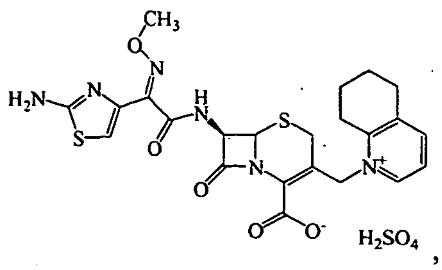

*i.e.*, 1-[[6R,7R)-7-[2-(2-amino-4-thiazolyl)glyoxylamido]-2-carboxy-8-oxo-5-thia-1-azabicyclo[4.2.0] oct-2-en-3-yl]methyl]-5,6,7,8-tetrahydroquinolinium hydroxide, inner salt $7^2$-(Z)-(O-methyloxime), sulfate. In this formulation, the cefquinome sulfate is suspended in ethyl oleate. It has been reported that intramuscular or subcutaneous injection of the COBACTAN 2.5%™ at a dose of 1 mg cefquinome sulfate per kg bodyweight generally results in an efficacious plasma concentration over a period of 8-12 hours. With such a dosage, treatment with daily injections for 3-5 consecutive days is recommended.

In U.S. Patent No. 5,071,979, Lattrell et al. discuss a genus of cephalosporin compounds, as well as methods for making such compounds and methods for using such compounds to treat bacterial infections. This genus includes cefquinome and physiologically acceptable acid addition salts thereof.

In U.S. Patent No. 4,845,087, Lattrell et al. discuss crystalline acid addition salts of cefquinome, and the use of such crystallized salts to treat bacterial infections. Lattrell et al. report that the crystallized salts exhibit antibacterial properties against both gram-positive and gram-negative bacterial germs. Lattrell et al. also report that the crystallized salts are unexpectedly active against penicillinase- and cephalosporinase-forming bacteria, and exhibit favorable toxicological and pharmacological properties, making them valuable chemotherapeutic agents.

In U.S. Patent No. 5,747,484, Lattrell et al. discuss a genus of phenolic carboxylic acid addition salts of cephalosporin compounds, as well as methods for making such compounds and methods for using such compounds to treat bacterial infections. This genus includes carboxylic acid addition salts of cefquinome. Lattrell et al. report that these salts provide advantages based on their low solubility and pharmacokinetics in animals.

In U.S. Patent No. 4,692,516, Kirrstetter et al. discuss a process for making a genus of 3-pyridinium-methylcephalosporins by nucleophilic replacement in the presence of tri-$C_1$-$C_4$-alkyl iodosilane. The cephalosporin genus

includes cefquinome.

In U.S. Patent No. 6,911,441, Schmid et al. discuss compositions of a cephalosporin (*e.g.*, cefquinome) in a release vehicle comprising an oil and aluminum distearate. Schmid et al. report that such compositions provide a prolonged duration of effective blood-plasma concentration of the cephalosporin after injection to an animal.

EP 0 388 510 describes a parenteral formulation comprising decaplanin and a cephalosporine derivative.

Despite the foregoing, there continues to be a need for alternative cefquinome formulations that, for example, enable consistent dosing, are simple to administer (*e.g.*, good syringeability), remain stable at ambient temperatures, are readily absorbed, have high local tolerability, have zero-day withholding, and/or have zero-day milk discard. The following disclosure describes such formulations and methods for using such formulations.

## SUMMARY OF THE INVENTION

This invention is related to cefquinome compositions and their use to treat infections in animals. Such compositions are particularly suitable to be used with mammals. Such mammals include, for example, swine, bovines, and equines. It is further contemplated that such mammals include, for example, other farm or livestock mammals (*e.g.*, goats, sheep, etc.), laboratory mammals (*e.g.*, mice, rats, etc.), companion mammals (*e.g.,* dogs, cats, etc.), and wild and zoo mammals (*e.g.*, buffalo, deer, etc.). It is contemplated that the compositions of this invention also are suitable for use with other animals, such as birds (*e.g.*, turkeys, chickens, etc.) and fish.

Briefly, therefore, this invention is directed, in part, to a liquid (particularly aqueous) composition suitable for parenteral administration to an animal. The composition comprises cefquinome or a pharmaceutically acceptable salt thereof. In addition, the composition comprises dibasic sodium phosphate (*i.e.*, $Na_2HPO_4$).

This invention also is directed, in part, to the use of an above-described liquid composition to prepare a medicament, particularly a medicament for treating a bacterial infection in an animal.

This invention also is directed, in part, to a method for treating a bacterial infection in an animal. The method comprises forming a liquid composition. The composition comprises cefquinome or a pharmaceutically acceptable salt thereof. In addition, the composition comprises dibasic sodium phosphate.

The invention also is directed, in part, to a kit. The kit comprises a first volume comprising a therapeutically effective amount of cefquinome or a pharmaceutically acceptable salt thereof. In addition, the kit comprises a second volume comprising water. Further, the kit comprises dibasic sodium phosphate or a hydrate thereof. The dibasic sodium phosphate or hydrate may be present in the first volume, the second volume, and/or a third volume.

Further benefits of Applicants' invention will be apparent to one skilled in the art from reading this specification.

## DETAILED DESCRIPTION OF PREFERRED EMBODIMENTS

This detailed description of preferred embodiments is intended only to acquaint others skilled in the art with Applicants' invention, its principles, and its practical application so that others skilled in the art may adapt and apply the invention in its numerous forms, as they may be best suited to the requirements of a particular use. This detailed description and its specific examples, while indicating preferred embodiments of this invention, are intended for purposes of illustration only. This invention, therefore, is not limited to the preferred embodiments described in this specification, and may be variously modified.

The compositions of this invention may be used to treat a gram-positive bacterial infection, a gram-negative bacterial infection, or both. In some embodiments, for example, the composition may be used to treat an infection by *Actinobacillus spp., Actinobacillus pleuropneumoniae, Haemophilus spp., Clostridium spp., Corynebacterium, Erysipelothrix rhusiopathiae, Streptococcus spp.,* and/or *Pasteurella spp.* Exemplifying further, the composition may be used to treat respiratory infections (*e.g., Mannheimia haemolytica* infections in bovine animals), foot infections, septicemia, meningitis caused by *Streptococcus suis,* epidermatitis caused by *Staphylococcus spp.,* and/or mastitis-metritis-agalactia syndrome caused by *E. coli* or *Staphylococcus spp.*

The compositions of this invention are generally intended to be administered parenterally, although other modes of administration are contemplated. "Parenteral administration" includes, for example, subcutaneous injections, intravenous injections, intramuscular injections, intrasternal injections, submucosal injections, and infusion.

Parenteral administration of cefquinome compounds may be achieved using an aqueous solution of cefquinome or a salt thereof. Such aqueous solutions, however, tend to be unstable over long periods. Thus, a preferred packaging strategy comprises using a container (*e.g.*, a vial) comprising the cefquinome active ingredient and a separate container (*e.g.*, a second vial) comprising the solvent. Shortly before administration, the user can mix the cefquinome active ingredient with the solvent to form the aqueous solution for parenteral administration. Although it is contemplated that free cefquinome may be used in accordance with this packaging strategy, use of a pharmaceutically acceptable salt of cefquinome is typically more preferred. More specifically, various salts of cefquinome tend to exhibit greater stability as a solid. Contemplated salts include, for example, cefquinome dihydrochloride, cefquinome sulfate, cefquinome-6-hydroxynaphtoate ("cefquinome-naphtoate"), and cefquinome-2,4-dihydroxybenzoate ("cefquinome hydroxybenzoate"). Cefquinome sulfate is generally preferred due to its crystalline properties, solubility in water, and stability profile.

In general, the composition is administered in a dosage that provides a therapeutically effective amount of cefquinome or a salt thereof to the recipient animal. This is particularly true where the cefquinome or salt thereof is the only active ingredient in the composition. To the extent the cefquinome or salt thereof is administered with another active ingredient(s), the dosage preferably comprises an amount of the cefquinome or salt thereof that, together with the amount of the other active ingredient(s), constitutes a therapeutically effective amount.

As used in this patent, the term "therapeutically effective amount" constitutes an amount that is sufficient to prevent, reduce the risk of, delay the onset of, ameliorate, suppress, or eradicate a target pathogen(s) infection. Generally, the therapeutically effective amount is defined as the amount necessary to achieve a concentration efficacious to control the target pathogen(s) at the site of infection (or, when used to prevent, reduce the risk of, or delay the onset of infection, at a site susceptible to infection). The concentration at the site of infection (or at a site susceptible to infection) is preferably at least equal to the $MIC_{90}$ level (minimum inhibitory concentration, *i.e.*, the concentration that inhibits the growth of 90% of the target pathogen) of the cefquinome or salt thereof for the target pathogen. For example, the $MIC_{90}$ for *Mannheimia haemolytica* is about 0.25 μg/ml.

Although it is contemplated that compositions of this invention may be dosed at a frequency of less than once per day (*e.g.*, every other day), the compositions are typically dosed at least once per day. The preferred total daily dose of the cefquinome or salt thereof is typically greater than about 0.5 mg/kg (*i.e.*, gram of cefquinome or salt thereof per kilogram body weight). In some embodiments, the daily dose is from about 1 to about 15 mg/kg. In some such embodiments, the daily dose is from about 1 to about 2 mg/kg. In other such embodiments, the daily dose is from about 5 to about 5 to 10 mg/kg. Although single daily doses are typically preferred, it is contemplated that dosage unit compositions may contain less than the total daily dose, and that such smaller doses are administered two or more times per day to achieve the desired total daily dose. For example, for foal septicemia, it may be preferable to administer two 1 mg/kg doses every 12 hours. It should be recognized that multiple doses per day may, in some instances, be used to increase the total daily dose, if desired.

Factors affecting the preferred dosage regimen include the type (*e.g.*, species and breed), age, weight, sex, diet, activity, and condition of the animal patient; the severity of the pathological condition; the apparatus used to administer the composition, as well as the type of parenteral administration used (*e.g.*, subcutaneous, intramuscular, submucosal); pharmacological considerations, such as the activity, efficacy, pharmacokinetic, and toxicology profiles of the particular composition administered; the existence of an additional active ingredient(s) in the composition; and whether the composition is being administered as part of a drug and/or vaccine combination. Thus, the dosage actually employed can vary for specific animal patients, and, therefore, can deviate from the typical dosages set forth above. Determining such dosage adjustments is generally within the skill of those in the art using conventional means. It is contemplated that the composition may be administered to the animal patient a single time. In general, however, the composition is administered daily for at least about 2 days, more typically daily for from about 3 to about 10 days, and still more typically daily for from about 3 to about 5 days.

The concentration of the cefquinome or salt thereof in the composition is preferably sufficient to provide the desired therapeutically effective amount of cefquinome or salt thereof in a volume that is acceptable for parenteral administration. The maximum acceptable volume may vary, depending on, for example, the apparatus being used for the administration, type of parenteral administration, size of the recipient animal, and subjective desires of the user. Generally, a parenteral dosage does not exceed about 100 ml, more typically about 75 ml, and more typically about 50 ml. In some embodiments, the preferred dosage volume is from about 5 to about 15 ml, or from about 5 to about 10 ml. Many adult horses, for example, weigh around 450 kg. With a 4.5% solution, the horse can be dosed with 1 mg/kg using 10 ml of the solution. Such a volume is often advantageous because it may typically be administered with one injection (rather than multiple injections), given the commercial availability of 10 ml syringes.

In general, the preferred concentration of the cefquinome or salt thereof in the composition is at least about 20 mg/ml, at least about 30 mg/ml, from about 40 to about 57 mg/ml, or from about 40 to about 55 mg/ml. In some embodiments, the concentration of the cefquinome or salt thereof in the composition is from about 44 to about 57 mg/ml. In some embodiments, active cefquinome mass is present in the composition at a concentration of from about 44 to about 48 mg/ml. In some embodiments, active cefquinome mass is present in the composition at a concentration of from about 44 to about 46 mg/ml. In some embodiments, cefquinome sulfate is present in the composition at a concentration of from about 52 to about 57 mg/ml. In some embodiments, cefquinome sulfate is present in the composition at a concentration of from about 52 to about 55 mg/ml. The concentration of the cefquinome or salt thereof preferably does not exceed the saturation concentration at ambient temperature (or the temperature at which the composition is administered).

In accordance with this invention, Applicants have discovered that a dibasic sodium phosphate solution can be advantageously used to reconstitute a cefquinome solid to form an aqueous composition for parenteral administration. Use of the dibasic sodium phosphate solution solves both the need to solubilize the cefquinome (or a salt thereof) and

the need to neutralize the cefquinome (or salt thereof). An aqueous solution comprising cefquinome or a salt thereof alone at concentrations contemplated by this invention is acidic. In the absence of a base, the pH for such a solution would be less than 7, and normally closer to about 1.5. It is preferred that sufficient dibasic sodium phosphate be present in the composition to impart a pH of at least about 4 to the composition. In some such embodiments, sufficient dibasic sodium phosphate is present to impart a pH of from about 4 to about 8 to the composition. In other such embodiments, sufficient dibasic sodium phosphate is present to impart a pH of from about 5 to about 7.5 to the composition. In other such embodiments, sufficient dibasic sodium phosphate is present to impart a pH of from about 6 to about 7 to the composition. In other such embodiments, sufficient dibasic sodium phosphate is present to impart a pH of from about 6.2 to about 6.7 to the composition. An example of a preferred pH for the composition is 6.3. Another example of a preferred pH for the composition is 6.5.

The preferred concentration of dibasic sodium phosphate may vary depending on, for example, whether cefquinome or a cefquinome salt is used; the type of salt, to the extent a salt is used; the concentration of the cefquinome or salt thereof; the preferred pH for the particular animal recipient; and the presence and concentration of any other active or inactive ingredient in the composition. Normally, the dibasic sodium phosphate concentration is at least about 100 mM, from about 150 to about 500 mM, from about 250 to about 400 mM, or from about 300 to about 350 mM.

It is contemplated that the dibasic sodium phosphate may be packaged separately from the cefquinome or salt thereof. In such embodiments, the cefquinome or salt is typically in the form of a solid, and the dibasic sodium phosphate is in the form of an aqueous solution that is used to reconstitute the cefquinome or salt thereof before administration. Such a dibasic sodium phosphate solution may be prepared by, for example, dissolving dibasic sodium phosphate itself ($Na_2HPO_4$) into water. Typically, however, a hydrate of dibasic sodium phosphate is instead dissolved into water to form the solution. Suitable hydrates may include, for example, the dihydrate ($Na_2HPO_4{}^{-}2H_2O$) and the heptahydrate ($Na_2HPO_4{}^{-}7H_2O$).

Although dibasic sodium phosphate is a preferred buffer, other buffers are contemplated either for use alone or in combination. Selection of a suitable buffer generally depends on factors such as, for example, the pH, osmolality, and stability imparted by the buffer upon the solvent and final composition for parenteral administration. Contemplated alternative buffers include, for example, sodium acetate, potassium phosphate monobasic, sodium phosphate monobasic, sodium bicarbonate, and sodium carbonate.

It is contemplated that the composition may comprise one or more conventional pharmaceutically acceptable carriers, vehicles, and/or adjuvants (collectively referred to as "excipients"), in addition to the cefquinome or salt thereof, dibasic sodium phosphate, and water. For example, although the compositions of this invention (as well as their individual components) are generally not susceptible to bacterial growth, it may be desirable in some instances for the composition (particularly the solvent) to comprise one or more preservatives. The presence of a preservative may, for example, provide a benefit for compositions or solvents that may be stored over lengthy periods of time, *e.g.*, days, weeks, months, or years. When selecting a suitable preservative, factors to consider include, for example, its antimicrobial activity (*e.g.*, against S. *aureus, P. aeruginosa, C. albicans*, and/or *A. niger);* the pH range at which it has the desired antimicrobial activity; the minimum concentration at which it has the desired antimicrobial activity; its aqueous solubility and other physical characteristics (*e.g.*, potential to cause foaming); its suitability for parenteral use; its possible interactions with the active ingredient(s) (*e.g.*, its effect on the solubility of an active ingredient); its possible interactions with the non-active ingredients (*e.g.*, its effect on the stability of the solvent); and any government regulations that may be applicable where the composition or solvent is being manufactured, sold, or used. Contemplated preservatives include, for example, parabens, propylene glycol, benzalkonium chloride, phenylethanol, chlorocresol, metacresol, ethanol, phenoxyethanol, and benzyl alcohol. Benzyl alcohol is generally preferred. The concentration of the preservative is typically greater than about 5 mg/ml. In some embodiments (*e.g.*, where the preservative is benzyl alcohol), the concentration is from about 5 to about 15 mg/ml, from 7.5 to about 15 mg/ml, from about 10 to about 15 mg/ml, or about 10 mg/ml. In some embodiments, the concentration is at least about 10 mg/ml. In general, the concentration of the preservative(s) is less than about 150 mg/ml, and more typically no greater than about 20 mg/ml.

The present invention further comprises kits that are suitable for use in performing the methods of treatment described above. The kit comprises a first dosage form comprising cefquinome or a pharmaceutically acceptable salt thereof (*e.g.*, cefquinome sulfate). The kit also comprises at least one additional component, and, typically, instructions for using the first dosage form with the additional component(s). The additional component(s) may, for example, be one or more additional ingredients that can be mixed with the first dosage form before or during administration. The additional component(s) may alternatively (or additionally) comprise one or more apparatuses for administering the first dosage form, additional pharmaceutical or biological materials, and/or diagnostic tools. The apparatus for administration may be, for example, a syringe, jet injector, or any other medically acceptable parenteral delivery vehicle.

In some preferred embodiments, the first dosage form comprises a solid (*e.g.*, powder) in a first container, and the second component comprises a solvent in a second container. The solvent preferably has sufficient chemical properties and quantity to solubilize the solid upon mixing. In these embodiments, it is generally desirable for the solid to dissolve in the solvent in less than 5 about minutes (or less than about 2 minutes, less than about 1 minute, or less than

about 30 seconds, or less than about 15 seconds) after mixing the solid with the solvent at 25°C. It also is desirable for the solvent to have chemical properties that allow the solid to remain dissolved in the solvent at 25°C for at least 5 minutes, at least 10 minutes, at least an hour, at least a day, at least a week, or at least a month.

The first and second containers may be attached or separate. Both containers preferably have a shape, size, composition, and cleanliness that are suitable for pharmaceutical use. Suitable containers may be, for example, vials having a volume of from about 30 to about 100 ml. Such vials are typically suitable for kits used for treating, for example, swine, bovines, and equines.

The container containing the solid (and, in some embodiments, the final composition containing the solid reconstituted into a solvent) preferably is suitable for acidic solutions (*i.e.*, a pH of less than 7) and slightly basic solutions. Such solutions generally have a pH of no greater than 7.5. Typical pH ranges are from about 1.5 to about 7.5, from about 5.5 to about 7.5, or from about 6.3 to about 6.5. In some embodiments, the container containing the solvent comprises a type II glass container.

The container containing the solvent preferably is suitable for basic solutions (*i.e.*, a pH of greater than 7), particularly in embodiments where the solvent comprises a dibasic sodium phosphate solution, which typically has a pH of at least about 8.0, from about 8.5 to about 9.5, or from about 9.1 to about 9.3. In some embodiments, the solvent container comprises a type I glass container. In other embodiments, the solvent container comprises a pharmaceutical grade resin. In such embodiments, for example, the container/solvent combination may be prepared via a continuous process using a blow-fill-seal apparatus in which the container is formed, filled with sterile solvent, and sealed in a single sterile, enclosed area without human intervention.

Kits comprising two containers preferably include a mechanism that enables the contents of one container to be transferred to the other for mixing without contamination. Where one container contains solid cefquinome (or a salt thereof) and another container contains a solvent, the kit may, for example, comprise a transfer spike (*e.g.*, a vented needle) that forms a part of, can be pierced through, or can be connected to (via, for example, a leur lock) the top of the solvent container. In such embodiments, the cap of the solid-containing container may comprise a resilient rubber stopper or other structure (*e.g.*, a bromobutyl stopper) that may be pierced by the spike, thereby allowing the solvent to flow from the solvent container into the solid-containing container for mixing. In the event that the solvent container is a soft resin material, the solvent may, for example, be forced into the solid-containing container by gently squeezing the solvent container.

Following removal of the spike, the solid preferably is permitted to dissolve in the solvent. The mixture may be agitated by, for example, gentle hand or arm motion to ensure that the solid completely dissolves. Once the solid has dissolved, the needle of a syringe apparatus can be inserted through the same rubber or other structure to withdraw the mixture from the container and into the syringe for parenteral administration to the animal patient.

EXAMPLES

The following examples are merely illustrative, and not limiting to the remainder of this disclosure in any way.

**Example 1. Example kit**

A kit can be prepared having the following components:

(1) a 100 ml, colorless, type II glass vial comprising 5.56 g cefquinome sulfate. This is equivalent to 4.5 g active cefquinome mass, taking into account the average purity (*i.e.*, 810 mg/g).

(2) a 100 ml, colorless, type I glass vial comprising a dibasic sodium phosphate solution prepared by combining 4.936 g $Na_2HPO_4{}^-2H_2O$, 0.96 g benzyl alcohol, and sufficient sterile water to bring the total volume in the vial to 96 ml.

An analogous kit having lesser quantities can similarly be prepared. For example, a kit can be prepared having the following components:

(1) a 30 ml, colorless, type II glass vial comprising 1.67 g cefquinome sulfate. This is equivalent to 1.35 g active cefquinome mass.

(2) a 30 ml, colorless, type I glass vial comprising a dibasic sodium phosphate solution prepared by combining 1.491 g $Na_2HPO_4{}^-2H_2O$, 0.29 g benzyl alcohol, and sufficient sterile water to bring the total volume in the vial to 29 ml.

**Example 2. Another example kit.**

A kit was prepared having the following components:

(1) a 100 ml, colorless, type II glass vial comprising 5.56 g cefquinome sulfate.

(2) a 100 ml, colorless, type I glass vial comprising a dibasic sodium phosphate solution prepared by combining 7.742 g $Na_2HPO_4{}^-7H_2O$ and sufficient sterile water to bring the total volume to 96 ml.

**Example 3. Withdrawal and Tolerance Study in Cattle.**

A study was conducted to determine the concentration of cefquinome residues in muscle and kidney tissue at a single time point at 12(±0.5) hr after daily subcutaneous administration of 2 mg active cefquinome mass per kilogram body weight for five consecutive days. In addition, assessment of injection site irritation was conducted.

*Animals.*

Seven crossbred Angus (commercial feedlot type; obtained from Gary Cope of Wellington, CO) calves were selected from an initial group of 11 calves, and randomly assigned to either a treatment or control group: (1) a treatment group of 3 castrated steers, (2) a treatment group of 3 non-pregnant intact heifers, and (3) a control group of 1 non-pregnant intact heifer. The animals were at approximately 8 months of age, and weighed from 184 to 208 kg at the start of the experiment.

Before beginning the treatments, the animals were vaccinated subcutaneously in axillary regions, avoiding any neck injections, with *Clostridium chauvoei, septicum, novyi, sordelli,* and *perfringens* C & D Bacterin-Toxoid (ELECTROID™ 7, serial no. 655C, exp. 05JUL2004, Schering Plough Animal Health); and killed viruses of bovine rhinotracheitis, bovine virus diarrhea, parainfluenza 3, and bovine respiratory syncytial virus (VIRASHIELD™ 5, serial no. 45-255C, exp. 07JUN04, Grand Laboratories, Inc.). In addition, an anthelmintic, ivermectin (PROMECTIN B™, lot no. 2030318, exp. 3/04, VEDCO) was applied as a pour-on. The calves were quarantined for at least 14 days, and then acclimated for at least an additional 7 days. No additional pharmaceuticals or antimicrobials were administered to the calves other than the cefquinome injections that were administered to the treated calves as discussed below.

The animals were housed as a commingled group, but segregated from other study animals under natural outdoor conditions in unbedded dirt-lot containment corrals. For the first 12 days (*i.e.*, until 11 days before the treatments began), the calves were given long-stem grass hay 2 times per day equivalent to approximately 3% of their body weight per day. Beginning on the seventh day (*i.e.*, 16 days before the treatments began), the animals also were given cracked corn equivalent to approximately one-half pound per calf per feeing. And beginning on the twelfth day, the twice-per-day regimen was changed to feeding long-stem grass hay at an amount approximately equivalent to 1.5% of the animals' body weight per day, plus a non-medicated calf concentrate (Calf Concentrate 32, Ranch-Way, Inc., Fort Collins, CO) at the rate of approximately 0.25 pound per calf per feeding. Each animal was observed for clinical abnormalities at least once per day, and examined by a veterinarian on the 7th day, and on the day before the start of the treatments. As of the day before the start of the treatments, all 7 calves were found to be healthy and exhibiting no abnormal signs or other abnormalities. The animals were weighed on the 16th day, and the day before the start of the treatments. Trace mineralized salt blocks (MORTON™ IOFIXT™ T-M) were available at all times. Fresh water also was available at all times.

During the treatment period, the animals were observed twice per day (once in the morning, and once in the afternoon) for mortality, morbidity, or other clinical abnormalities. Body weights were determined on the first day of treatment and the sixth day.

*Preparation of cefquinome parenteral formulation.*

A reconstitutable cefquinome sulfate powder (G.C. Hanford Mfg. Co. (Syracuse, NY, USA)) in glass vials was used in this experiment. Each vial contained 4.5 active cefquinome mass in the form of a sterile, dry powder having a Certificate of Analysis ("C of A") of 846 mg/g cefquinome (102% of label claim). These vials were stored at 2-8°C.

A 300 mM dibasic sodium phosphate diluent (Vital Pharma, Inc., Riveria Beach, FL, USA)) in soft plastic vials was used to reconstitute the cefquinome sulfate powder. The diluent had a pH of 9.0, and a C of A of 41.8 mg/ml disodium phosphate. This diluent was stored at ambient temperatures of 62-76°F.

Lateral flow vented needles from B. Braun Medical Inc. (Bethlehem, PA, USA; Melsungen, Germany) were used to transfer the diluent into the powder vials for mixing. Before use, these needles were stored within plastic sealed paper packets.

The solid cefquinome powder was reconstituted with the diluent on the day of administration. The reconstituted solution was intended to contain an active cefquinome mass concentration of 45 mg/ml, although assayed samples of the reconstituted formulations were observed to be from 47 to 50 mg/ml (*i.e.*, from 4 to 10% greater), and on average 48 mg/ml (*i.e.*, 6% greater). Reconstituted formulations were held at ambient temperature in the vials or pre-filled syringes in a lidded insulated container for transportation to the animal dosing facility.

*Dosing.*

Each animal's dose was based on its day-1 body weight. The target dose concentration was 2 mg active cefquinome mass per kg of body weight. Thus, based on the estimated active cefquinome mass concentration being 45 mg/ml in the reconstituted formulation, the amount administered per day to each treated animal was calculated as follows:

$$\text{volume of reconstituted cefquinome formulation administered} = \frac{\text{body weight x 2 (mg/kg)}}{\text{45 (mg active cefquinome mass/ml)}}$$

The dose volume was injected at an accuracy of 0.2 ml using 12 ml syringes with 0.2 ml graduations. Dose volumes were rounded up to the nearest 0.2 ml (*e.g.*, a calculated does volume of 7.11 ml would be rounded up to 7.2 ml). The needles on the syringes were 16 ga, 0.75 inches. Body weights were such that only a single injection of <10 ml was required per day per calf.

The control calf received no injections. As to the treated calves, on each of study days 1-5, the 3 steers and 3 heifers were dosed subcutaneously in the neck to achieve approximately 2 mg active cefquinome mass per kg body weight. Dosing sites were alternated left to right across days (day 1, site 1: left anterior; day 2, site 2: right anterior; day 3, site 3: left middle; day 4, site 4: right posterior, and day 5, site 5: left posterior). Injections were administered at approximately the same time on all treatment days.

*Tissue collection.*

Each of the six treated calves was humanely euthanized at 11.5 hr after its fifth treatment (within protocol tolerance of 12 $\pm$0.5 hr). The control calf was humanely euthanized before the 12-hour termination time of the treated animals.

Muscle tissue was collected from the left and right triceps and left and right longissimus dorsi of each calf. To achieve 1:1 proportional composite samples of triceps and L. dorsi that were approximately 0.5 kg per body side, the four pieces were trimmed to approximately 250 g and weights recorded. The left triceps sample was combined with the left L. dorsi sample, and the combination was used as a retention sample. The right triceps and right L. dorsi samples were combined and used for residue concentration analysis.

Both the left and right kidneys were collected and trimmed of fat, capsular material, and uteters, and then weighed. The left kidney was cut longitudinally in half. One half was used for Fast Antimicrobial Screen Test (FAST) assaying purposes. The right kidney was used for residue concentration analysis.

The thoracic and abdominal organs and cavities of each calf were examined by a board certified pathologist for pathology and other abnormalities. In addition, injection sites were evaluated by a certified pathologist.

*Results.*

The control calf had no detectible cefquinome levels in the kidney or muscle (limits of detection 0.200 ppm and 0.0650 ppm, respectively). Cefquinome was not detectable from the muscle of any treated calf. Kidney cefquinome concentrations in the treated calves ranged from 1.69 ppm to 2.83 ppm. All treated-calf tissues were therefore below 1.34 ppm for muscle and 7.82 ppm for kidney at 12 hr after final treatment, thus supporting a zero withdrawal period.

Among all the calves, the only finding of gross pathology of the organs of the abdomen and thorax was a pendulous mass of 2x5 cm in the pelvic cavity of one treated steer. This mass was determined to be a necrotic tissue encapsulated by fibrous connective tissue and of long duration, thus not being related to study activities.

During antemortem analysis of injection sites, only one calf exhibited a reaction at an injection site. That calf exhibited a soft swelling (6 cm long, 4 cm wide, 1 cm high) at the site of its fifth injection one day after the injection. No other reactions of heat, swelling, or pain were exhibited by any calf.

During postmortem analysis of injection sites, only a single abnormality (induration, 5 x 6, mild) was observed in the 30 sites examined as outer, non-incised skin surfaces. Abnormalities, however, were observed in 28 of the 30 sites examined, both as *in situ* skin undersurface with underlying tissue and as excised knife-cute cores. These were primarily hemorrhage, edema, necrosis, and inflammatory cell infiltration in the subcutaneous connective tissues. It was determined, however, that the majority of these abnormalities were minimal to mild reversible lesions, and that the subcutaneous administration of the cefquinome formulation at 2mg/kg for 5 days was well-tolerated.

The term "pharmaceutically acceptable" is used adjectivally in this patent to mean that the modified noun is appropriate for use in a pharmaceutical product. When it is used, for example, to describe an excipient in a pharmaceutical composition, it characterizes the excipient as being compatible with the other ingredients of the composition and not disadvantageously deleterious to the intended recipient animal.

The words "comprise", "comprises", and "comprising" in this patent (including the claims) are to be interpreted inclusively rather than exclusively. This interpretation is intended to be the same as the interpretation that these words are given under United States patent law.

## Claims

1. A liquid composition suitable for parenteral administration to a mammal, fish, or bird, **characterized in that** the

composition comprises:

a therapeutically effective amount of cefquinome or a pharmaceutically acceptable salt thereof, and
dibasic sodium phosphate.

2. The composition according to claim 1, **characterized in that** the cefquinome or salt thereof is present in the composition at a concentration of from 44 to 57 mg/ml.

3. The composition according to claim 1 or 2, **characterized in that** the dibasic sodium phosphate is present in the composition at a concentration that is sufficient to impart a pH of from 6 to 7 to the composition.

4. The composition according to any one of claims 1 to 3, **characterized in that** the composition comprises benzyl alcohol.

5. The composition according to any one of claims 1 to 4, **characterized in that** the composition comprises cefquinome sulfate.

6. The composition according to any one of claims 1 to 5, **characterized in that** the composition comprises: cefquinome sulfate at a concentration of from 52 to 57 mg/ml, and dibasic sodium phosphate at a concentration of from 300 to 350 mM.

7. Use of a composition according to any one of claims 1 to 6 for the manufacture of a medicament for treating a bacterial infection.

8. A kit, wherein the kit comprises:

a first container comprising a therapeutically effective amount of cefquinome or a pharmaceutically acceptable salt thereof,
a second container comprising water, and
dibasic sodium phosphate or a hydrate thereof.

9. The kit according to claim 8, wherein the first container comprises a therapeutically effective amount of cefquinome sulfate.

10. The kit according to claim 8 or 9, wherein dibasic sodium phosphate is present in the kit as an aqueous dibasic sodium phosphate solution in the second container.

11. The kit according to any one of claims 8 to 10, wherein the kit further comprises an apparatus for parenterally administering a mixture of the cefquinome (or salt thereof) and dibasic sodium phosphate solution to a mammal.

12. The kit according to claim 11, wherein the apparatus comprises a syringe.

13. The kit according to claim 11 or 12, wherein the mammal is a bovine, equine, or swine animal.

## Patentansprüche

1. Flüssige Zusammensetzung, die für die parenterale Verabreichung an einen Säuger, Fisch oder Vogel geeignet ist, **dadurch gekennzeichnet, dass** die Zusammensetzung umfasst:

eine therapeutisch wirksame Menge Cefquinom oder eines pharmazeutisch verträglichen Salzes davon, und
dibasisches Natriumphosphat.

2. Zusammensetzung gemäß Anspruch 1, **dadurch gekennzeichnet, dass** das Cefquinom oder das Salz davon in der Zusammensetzung mit einer Konzentration von 44 bis 57 mg/ml vorhanden ist.

3. Zusammensetzung gemäß Anspruch 1 oder 2, **dadurch gekennzeichnet, dass** das dibasische Natriumphosphat in der Zusammensetzung mit einer Konzentration vorhanden ist, die ausreicht, um der Zusammensetzung einen

pH-Wert von 6 bis 7 zu verleihen.

4. Zusammensetzung gemäß einem der Ansprüche 1 bis 3, **dadurch gekennzeichnet, dass** die Zusammensetzung Benzylalkohol umfasst.

5. Zusammensetzung gemäß einem der Ansprüche 1 bis 4, **dadurch gekennzeichnet, dass** die Zusammensetzung Cefquinomsulfat umfasst.

6. Zusammensetzung gemäß einem der Ansprüche 1 bis 5, **dadurch gekennzeichnet, dass** die Zusammensetzung umfasst: Cefquinomsulfat mit einer Konzentration von 52 bis 57 mg/ml und dibasisches Natriumphosphat mit einer Konzentration von 300 bis 350 mM.

7. Verwendung einer Zusammensetzung gemäß einem der Ansprüche 1 bis 6 für die Herstellung eines Medikaments zum Behandeln einer bakteriellen Infektion.

8. Kit, wobei das Kit umfasst:

   einen ersten Behälter, umfassend eine therapeutisch wirksame Menge Cefquinom oder eines pharmazeutisch verträglichen Salzes davon,
   einen zweiten Behälter, umfassend Wasser, und
   dibasisches Natriumphosphat oder ein Hydrat davon.

9. Kit gemäß Anspruch 8, wobei der erste Behälter eine therapeutisch wirksame Menge Cefquinomsulfat umfasst.

10. Kit gemäß Anspruch 8 oder 9, wobei in dem Kit dibasisches Natriumphosphat als wässrige Lösung von dibasischem Natriumphosphat in dem zweiten Behälter vorhanden ist.

11. Kit gemäß einem der Ansprüche 8 bis 10, wobei das Kit ferner eine Vorrichtung zum parenteralen Verabreichen eines Gemischs des Cefquinom (oder des Salzes davon) und der Lösung von dibasischem Natriumphosphat an einen Säuger umfasst.

12. Kit gemäß Anspruch 11, wobei die Vorrichtung eine Spritze umfasst.

13. Kit gemäß Anspruch 11 oder 12, wobei der Säuger ein Rinder-, Pferde- oder Schweinetier ist.

## Revendications

1. Composition liquide adaptée à l'administration par voie parentérale à un mammifère, un poisson ou un oiseau, **caractérisée en ce que** la composition comprend :

   une quantité thérapeutiquement efficace de cefquinome ou d'un sel pharmaceutiquement acceptable de celle-ci, et
   du phosphate de sodium dibasique.

2. Composition selon la revendication 1, **caractérisée en ce que** la cefquinome ou un sel de celle-ci est présent(e) dans la composition à une concentration de 44 à 57 mg/ml.

3. Composition selon la revendication 1 ou 2, **caractérisée en ce que** le phosphate de sodium dibasique est présent dans la composition à une concentration qui est suffisante pour donner à la composition un pH de 6 à 7.

4. Composition selon l'une quelconque des revendications 1 à 3, **caractérisée en ce que** la composition comprend de l'alcool benzylique.

5. Composition selon l'une quelconque des revendications 1 à 4, **caractérisée en ce que** la composition comprend du sulfate de cefquinome.

6. Composition selon l'une quelconque des revendications 1 à 5, **caractérisée en ce que** la composition comprend :

du sulfate de cefquinome à une concentration de 52 à 57 mg/ml, et du phosphate de sodium dibasique à une concentration de 300 à 350 mM.

7. Utilisation d'une composition selon l'une quelconque des revendications 1 à 6, pour la fabrication d'un médicament destiné au traitement d'une infection bactérienne.

8. Kit, **caractérisé en ce que** le kit comprend :

   un premier récipient comprenant une quantité thérapeutiquement efficace de cefquinome ou un sel pharmaceutiquement acceptable de celle-ci,
   un deuxième récipient comprenant de l'eau, et
   du phosphate de sodium dibasique ou un hydrate de celui-ci.

9. Kit selon la revendication 8, **caractérisé en ce que** le premier récipient comprend une quantité thérapeutiquement efficace de sulfate de cefquinome.

10. Kit selon la revendication 8 ou 9, **caractérisé en ce que** le phosphate de sodium dibasique est présent dans le kit sous forme d'une solution aqueuse de phosphate de sodium dibasique dans le deuxième récipient.

11. Kit selon l'une quelconque des revendications 8 à 10, **caractérisé en ce que** le kit comprend en outre un appareil pour l'administration par voie parentérale d'un mélange de cefquinome (ou d'un sel de celle-ci) et d'une solution de phosphate de sodium dibasique à un mammifère.

12. Kit selon la revendication 11, **caractérisé en ce que** l'appareil comprend une seringue.

13. Kit selon la revendication 11 ou 12, **caractérisé en ce que** le mammifère est un animal bovin, équin ou porcin.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 73146305 P
- EP 06111029 A
- US 5071979 A, Lattrell
- US 4845087 A, Lattrell
- US 5747484 A, Lattrell
- US 4692516 A, Kirrstetter
- US 6911441 B, Schmid
- EP 0388510 A